# EUROPEAN PATENT SPECIFICATION

(11) **EP 3 325 460 B1**
(45) Date of publication and mention of the grant of the patent: **08.01.2020**
(21) Application number: 16745355.4
(22) Date of filing: 21.07.2016
(51) Int. Cl.: C07D 307/68

(54) **REACTIVE DISTILLATION PROCESS FOR THE ESTERIFICATION OF FURANDICARBOXYLIC ACID**
REAKTIVES DESTILLATIONSVERFAHREN ZUR VERESTERUNG VON FURANDICARBONSÄURE
PROCÉDÉ DE DISTILLATION RÉACTIVE POUR L'ESTÉRIFICATION DE L'ACIDE FURANE DICARBOXYLIQUE

(30) Priority: 24.07.2015 US 201562196812 P
(43) Date of publication of application: 30.05.2018
(73) Proprietor: DuPont Industrial Biosciences USA, LLC, Wilmington, Delaware 19805 (US)
(72) Inventor: METKAR, Pranit S., Wilmington, Delaware 19809 (US); SENGUPTA, Sourav Kumar, Wilmington, Delaware 19808 (US)
(74) Representative: Mewburn Ellis LLP
(86) International application number: PCT/US2016/043325
(87) International publication number: WO 2017/019447

(56) References cited:
- EP-A1- 1 849 764
- EP-A1- 2 481 733

## Description

### FIELD OF THE DISCLOSURE

The present disclosure is directed towards processes for making a dialkyl ester of 2,5-furan dicarboxylic acid (FDCA) using reactive distillation.

### BACKGROUND OF THE DISCLOSURE

Poly(trimethylene furandicarboxylate) (PTF) is a renewable polyester synthesized via the condensation reaction of 2,5-furandicarboxylic acid (FDCA) or 2,5-furandicarboxyl methyl ester (FDME) with 1,3 propane diol. Compared to poly(ethylene terephthalate) (PET), PTF demonstrates increased oxygen and CO₂ barrier properties that are attractive for carbonated-soft drink (CSD) and food packaging applications. The quality of monomer (especially color) is very important for obtaining a high quality colorless polymer required for beverage, food and packaging industry. The use of FDME instead of FDCA as a monomer usually results in better color for the final PTF polymer.

Currently, FDCA and FDME are not manufactured at commercial scale. For the production of FDME, one option is to use a process similar to the process for the conversion of terephthalic acid (TPA) to dimethyl terephthalate (DMT), which has been used commercially for many years. However, thermal stability of FDCA and TPA are very different and the FDCA molecule is not expected to be stable at 305 °C, so a lower temperature reaction would be preferred. However, lower temperature reaction may not be commercially viable since the reaction rate ofuncatalyzed FDME synthesis at lower temperature may be too slow.

Furthermore, the quality of the resulting monomer may not be suitable for the production of PTF polymer.

EP 2481733 A1 teaches the formation of esters of 2,5-FDCA by reacting the compound with one or more alcohols in the presence of a heterogeneous catalyst. In the specific examples, reactions are carried out in a stirred reactor at temperatures between 194°C and 246°C.

EP 1849764 A1 describes a specific process for the continuous esterification of an organic acid with two or three acid groups to produce an organic acid di- or tri-ester with the available acid groups esterified in a vertical column by reactive distillation. The application is specifically concerned with esterification of citric acid.

In view of the above, there is a need for a new process for the production of a dialkyl ester of FDCA.

### SUMMARY OF THE DISCLOSURE

In a first embodiment, there is a process comprising:
a) providing a reactive distillation column comprising a top, a bottom, and a reaction zone in between the top and the bottom, and a solid acid catalyst situated in the reaction zone;
b) contacting a feed comprising 2,5-furan dicarboxylic acid (FDCA) and a high boiling solvent with an alcohol in the reaction zone at a temperature in the range of 40 °C to 300 °C and a pressure in the range of 0.0007 MPa to 3.4 MPa for a residence time sufficient to produce a mixture comprising a dialkyl ester of FDCA and water;
c) removing a vapor mixture comprising the alcohol and water from the top of the reactive distillation column;
d) collecting a product stream comprising the dialkyl ester of FDCA, the high boiling solvent, and optionally one or more of a monoalkyl ester of FDCA, any unreacted FDCA and any other high boilers, from the bottom of the reactive distillation column; and
e) separating the dialkyl ester of FDCA from the product stream using distillation.

In a second embodiment of the process, the high boiling solvent comprises at least one of dimethyl sulfoxide, dimethylacetamide, sulfolane, FDME, gamma-valerolactone, gamma-butyrolactone, isosorbide dimethyl ether, propylene carbonate, adipic acid, isosorbide, isophorone, ethyl phenyl ether, diphenyl ether, dibenzyl ether, aromatic 200 fluid, butyl phenyl ether, methyl heptyl ketone, ethyl phenyl ketone, 2'-hydroxyacetophenone, decahydronaphthalene, tetrahydronaphthalene, and the like.

In a third embodiment of the process, the alcohol comprises C₁ to C₆ alcohol.

In a fourth embodiment of the process, the alcohol is methanol.

In a fifth embodiment of the process, the alcohol is fed with a carrier gas from the bottom of the reactive distillation column and the feed comprising FDCA and the high boiling solvent is fed from the top of the reactive distillation column.

In a sixth embodiment of the process, the acid catalyst comprises a heterogeneous heteropolyacid, a salt of a heterogeneous heteropolyacid, a natural or synthetic mineral (including both clays and zeolites), an ion-exchange resin, a metal oxide, a mixed metal oxide, a metal sulfide, a metal sulfate, a metal sulfonate, sulfated titania, sulfated zirconia, a metal nitrate, a metal phosphate, a metal phosphonate, a metal molybdate, a metal tungstate, a metal borate, sulfonic-acid-functionalized polymer, or a combination of any of these.

In a seventh embodiment of the process, the acid catalyst comprises a zeolite.

In an eighth embodiment of the process, the acid catalyst is a medium or large pore, acidic, hydrophobic zeolite.

In a ninth embodiment of the process, the zeolite comprises ZSM-5, faujasite, beta zeolite, mordenite, or a combination of any of these.

In a tenth embodiment, the process comprises:
a) providing a reactive distillation column comprising a top, a bottom, and a reaction zone in between the top and the bottom, and a solid acid catalyst situated in the reaction zone;
b) contacting a feed comprising 2,5-furan dicarboxylic acid (FDCA) and a high boiling solvent with an alcohol in the reaction zone at a temperature in the range of 40 °C to 300 °C and a pressure in the range of 0.0007 MPa to 3.4 MPa for a residence time sufficient to produce a vapor mixture of a dialkyl ester of FDCA, water and optionally, a monoalkyl ester of FDCA;
c) removing the alcohol and the vapor mixture comprising the dialkyl ester of FDCA, water, and optionally the monoalkyl ester of FDCA from the top of the reactive distillation column;
d) collecting the high boiling solvent, optionally one or more of the monoalkyl ester of FDCA, any unreacted FDCA, and any other high boilers from the bottom of the reactive distillation column; and
e) separating the dialkyl ester of FDCA from the vapor mixture using distillation.

In an eleventh embodiment, the process comprises:
a) providing a reactive distillation column comprising a top, a bottom, and a reaction zone in between the top and the bottom;
b) contacting a feed comprising 2,5-furan dicarboxylic acid (FDCA), a high boiling solvent, and optionally a homogeneous catalyst, with an alcohol in the reaction zone in the absence of a solid acid catalyst at a temperature in the range of 150 °C to 300 °C and a pressure in the range of 0.0007 MPa to 3.4 MPa for a residence time sufficient to produce a mixture comprising a dialkyl ester of FDCA and water;
c) removing a vapor mixture comprising the alcohol and water from the top of the reactive distillation column;
d) collecting a product stream comprising the dialkyl ester of FDCA, the high boiling solvent, and optionally one or more of the homogeneous catalyst, a monoalkyl ester of FDCA, any unreacted FDCA and any other high boilers from the bottom of the reactive distillation column; and
e) separating the dialkyl ester of FDCA from the product stream using distillation.

In a twelfth embodiment of the process, the homogeneous catalyst is present, and the homogeneous catalyst is at least one of cobalt (II) acetate, iron (II) chloride, iron (III) chloride, iron (II) sulfate, iron (III) sulfate, iron (II) nitrate, iron (III) nitrate, iron (II) oxide, iron (III) oxide, iron (II) sulfide, iron (III) sulfide, iron (II) acetate, iron (III) acetate, magnesium (II) acetate, magnesium (II) hydroxide, manganese (II) acetate, phosphoric acid, sulfuric acid, zinc (II) acetate, zinc stearate or a combination thereof.

In a thirteenth embodiment, there is a process comprising:
a) providing a reactive distillation column comprising a top, a bottom, and a reaction zone in between the top and the bottom;
b) contacting a feed comprising 2,5-furan dicarboxylic acid (FDCA), a high boiling solvent, and optionally a homogeneous catalyst with an alcohol in the reaction zone in the absence of a solid acid catalyst at a temperature in the range of 150 °C to 300 °C and a pressure in the range of 0.0007 MPa to 3.4 MPa for a residence time sufficient to produce a mixture comprising a dialkyl ester of FDCA and water;
c) removing a vapor mixture comprising the dialkyl ester of FDCA, the alcohol, water, and optionally a monoalkyl ester of FDCA from the top of the reactive distillation column;
d) collecting the high boiling solvent, and optionally one or more of the homogeneous catalyst, the monoalkyl ester of FDCA, any unreacted FDCA and high boilers from the bottom of the reactive distillation column; and
e) separating the dialkyl ester of FDCA from the vapor mixture using distillation.

### BRIEF DESCRIPTION OF THE DRAWINGS

The invention is illustrated by way of example and not limited to the accompanying figures.
FIG. 1 is a schematic illustration of an exemplary reactor configuration used in the production of dialkyl ester of FDCA using a high boiling solvent in the presence of a solid acid catalyst in accordance with various embodiments of the processes disclosed herein.
FIG. 2 is a schematic illustration of another exemplary reactor configuration used in the production of dialkyl ester of FDCA using a high boiling solvent in the presence of a solid acid catalyst in accordance with various embodiments of the processes disclosed herein.
FIG. 3 is a schematic illustration of an exemplary reactor configuration used in the production of dialkyl ester of FDCA using a high boiling solvent and a homogeneous catalyst in absence of a solid acid catalyst in accordance with various embodiments of the processes disclosed herein.
FIG. 4 is another schematic illustration of an exemplary reactor configuration used in the production of dialkyl ester of FDCA using a high boiling solvent and a homogeneous catalyst in absence of a solid acid catalyst in accordance with various embodiments of the processes disclosed herein.

### DETAILED DESCRIPTION OF THE DISCLOSURE

The disclosures of all patent and non-patent literature cited herein are hereby incorporated by reference in their entirety.

The terms "comprises," "comprising," "includes," "including," "has," "having" or any other variation thereof, as used herein are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present). The phrase "one or more" is intended to cover a non-exclusive inclusion. For example, one or more of A, B, and C implies any one of the following: A alone, B alone, C alone, a combination of A and B, a combination of B and C, a combination of A and C, or a combination of A, B, and C.

Also, use of "a" or "an" are employed to describe elements and described herein. This is done merely for convenience and to give a general sense of the scope of the invention. This description should be read to include one or at least one and the singular also includes the plural unless it is obvious that it is meant otherwise.

The term "biologically-derived" as used herein is used interchangeably with "biobased" or "bio-derived" and refers to chemical compounds including monomers and polymers, that are obtained in whole or in any part, from any renewable resources including but not limited to plant, animal, marine materials or forestry materials. The "biobased content" of any such compound shall be understood as the percentage of a compound's carbon content determined to have been obtained or derived from such renewable resources.

The term "furandicarboxylic acid" as used herein is used interchangeably with furanoic acid; 2,5-furandicarboxylic acid; 2,4-furandicarboxylic acid; 3,4-furandicarboxylic acid; and 2,3-furandicarboxylic acid. As used herein, the 2,5-furandicarboxylic acid (FDCA), is also known as dehydromucic acid, and is an oxidized furan derivative, as shown below:

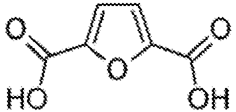

The acronym FDME means the dimethyl ester of 2,5-furan dicarboxylic acid.

The acronym FDMME means the monomethyl ester of 2,5-furan dicarboxylic acid.

The acronym FFME means the methyl ester of 5-formylfuran-2-carboxylic acid.

The term "solid acid catalyst" as used herein refers to any solid material containing Bronsted and/or Lewis acid catalytic sites, and which is substantially undissolved by the reaction medium under ambient conditions.

The term "high boiling solvent" as used herein denotes a solvent having a boiling point above about 100°C at one atmosphere.

The term "weight hourly space velocity" as used herein is used interchangeably with "WHSV" and is defined as the ratio of the mass flow rate of the reactants to the mass of the catalyst in the reactor.

Disclosed herein is a process of making a dialkyl ester of 2,5-furan dicarboxylic acid (FDCA) by contacting a feed comprising FDCA and a high boiling solvent with an alcohol in the presence of a solid acid catalyst using reactive distillation.

The process comprises a first step of providing a reactive distillation column comprising a top, a bottom, and a reaction zone in between the top and the bottom, and a solid acid catalyst situated in the reaction zone.

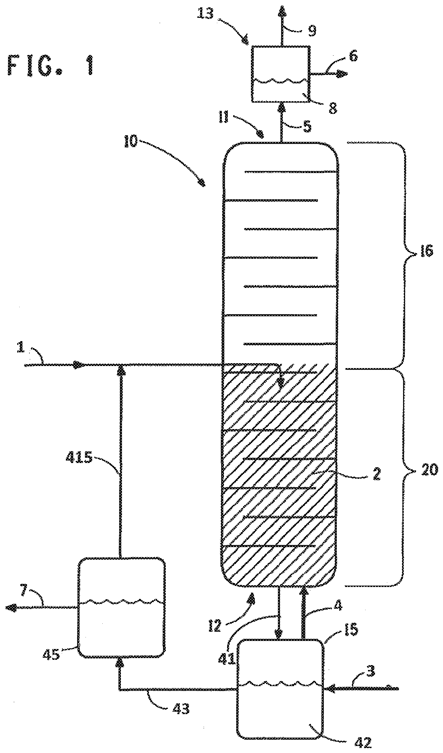

Figure 1 shows a schematic illustration of an exemplary reactor configuration comprising a reactive distillation column 10 comprising a top 11, a bottom 12, a reaction zone 20 in between the top 11 and the bottom 12, and a solid acid catalyst 2 disposed in the reaction zone 20.

The solid acid catalyst must have the thermal stability required to survive the reaction conditions and may be supported on at least one catalyst support. Examples of suitable solid acids include without limitation the following categories:
1) heterogeneous heteropolyacids (HPAs) and their salts,
2) natural or synthetic minerals (including both clays and zeolites), such as those containing alumina and/or silica,
3) metal oxides,
4) mixed metal oxides,
5) metal salts such as metal sulfides, metal sulfates, metal sulfonates, metal nitrates, metal phosphates, metal phosphonates, metal molybdates, metal tungstates, metal borates,
6) ion-exchange resins
7) sulfonic-acid-functionalized polymer, and
8) combinations of any members of any of these categories.

The metal components of categories 3 to 5 may be selected from elements from Groups 1 through 12 of the Periodic Table of the Elements, as well as aluminum, chromium, tin, titanium, and zirconium. Examples include, without limitation, sulfated zirconia and sulfated titania.

Suitable HPAs include compounds of the general formula XₐM_{b}O_{c}^{q-}, where X is a heteroatom such as phosphorus, silicon, boron, aluminum, germanium, titanium, zirconium, cerium, cobalt or chromium, M is at least one transition metal such as tungsten, molybdenum, niobium, vanadium, or tantalum, and q, a, b, and c are individually selected whole numbers or fractions thereof. Nonlimiting examples of salts of HPAs include, for example, lithium, sodium, potassium, cesium, magnesium, barium, copper, gold and gallium, and ammonium salts. Examples of HPAs suitable for the disclosed process include, but are not limited to, tungstosilicic acid (H₄[SiWi₂O₄₀]·xH₂O), tungstophosphoric acid (H₃[PW₁₂O₄₀]·xH₂O), molybdophosphoric acid (H₃[PMo₁₂O₄₀]·xH₂O), molybdosilicic acid (H₄[SiMo₁₂O₄₀]·xH₂O), vanadotungstosilicic acid (H₄₊ₙ[SiVₙW₁₂₋ₙO₄₀]·xH₂O), vanadotungstophosphoric acid (H₃₊ₙ[PVₙW₁₂₋ₙO₄₀]·xH₂O), vanadomolybdophosphoric acid (H₃₊ₙ[PVₙMo₁₂₋ₙO₄₀]·xH₂O), vanadomolybdosilicic acid (H₄₊ₙ[SiVₙMo₁₂₋ₙO₄₀]·xH₂O), molybdotungstosilicic acid (H₄[SiMoₙW₁₂₋ₙO₄₀]·xH₂O), molybdotungstophosphoric acid (H₃[PMoₙW₁₂₋ₙO₄₀]·xH₂O), wherein n in the formulas is an integer from 1 to 11 and x is an integer of 1 or more.

In one embodiment, the solid acid catalyst comprises natural or synthetic minerals (including both clays and zeolites), such as those containing alumina and/or silica.

Natural clay minerals are well known in the art and include, without limitation, kaolinite, bentonite, attapulgite, and montmorillonite.

Zeolites suitable for use herein can be generally represented by the following formula M_{2/n}O·Al₂O₃·xSiO₂·yH₂O wherein M is a cation of valence n, x is greater than or equal to about 2, and y is a number determined by the porosity and the hydration state of the zeolite, generally from about 2 to about 8. In naturally occurring zeolites, M is principally represented by Na, Ca, K, Mg and Ba in proportions usually reflecting their approximate geochemical abundance. The cations M are loosely bound to the structure and can frequently be completely or partially replaced with other cations by conventional ion exchange.

The zeolite framework structure has corner-linked tetrahedra with Al or Si atoms at centers of the tetrahedra and oxygen atoms at the corners. Such tetrahedra are combined in a well-defined repeating structure comprising various combinations of 4-, 6-, 8-, 10-, and 12-membered rings. The resulting framework structure is a pore network of regular channels and cages that is useful for separation. Pore dimensions are determined by the geometry of the aluminosilicate tetrahedra forming the zeolite channels or cages, with nominal openings of about 0.26 nm for 6-member rings, about 0.40 nm for 8-member rings, about 0.55 nm for 10-member rings, and about 0.74 nm for 12-member rings (these numbers assume the ionic radii for oxygen). Zeolites with the largest pores, being 8-member rings, 10-member rings, and 12-member rings, are frequently considered small, medium and large pore zeolites, respectively.

In a zeolite, the term "silicon to aluminum ratio" or, equivalently, "Si/AI ratio" means the ratio of silicon atoms to aluminum atoms. Pore dimensions are critical to the performance of these materials in catalytic and separation applications, since this characteristic determines whether molecules of certain size can enter and exit the zeolite framework.

It has been observed that very slight decreases in ring dimensions can effectively hinder or block movement of particular molecular species through the zeolite structure. The effective pore dimensions that control access to the interior of the zeolites are determined not only by the geometric dimensions of the tetrahedra forming the pore opening, but also by the presence or absence of ions in or near the pore. For example, in the case of zeolite type A, access can be restricted by monovalent ions, such as Na⁺ or K⁺, which are situated in or near 8-member ring openings as well as 6-member ring openings. Access can be enhanced by divalent ions, such as Ca²⁺, which are situated only in or near 6-member ring openings. Thus, the potassium and sodium salts of zeolite A exhibit effective pore openings of about 0.3 nm and about 0.4 nm respectively, whereas the calcium salt of zeolite A has an effective pore opening of about 0.5 nm.

The presence or absence of ions in or near the pores, channels and/or cages can also significantly modify the accessible pore volume of the zeolite for sorbing materials. Representative examples of zeolites are (i) small pore zeolites such as NaA (LTA), CaA (LTA), Erionite (ERI), Rho (RHO), ZK-5 (KFI) and chabazite (CHA); (ii) medium pore zeolites such as ZSM-5 (MFI), ZSM-11 (MEL), ZSM -22 (TON), and ZSM-48 (*MRE); and (iii) large pore zeolites such as zeolite beta (BEA), faujasite (FAU), mordenite (MOR), zeolite L (LTL), NaX (FAU), NaY (FAU), DA-Y (FAU) and CaY (FAU). The letters in parentheses give the framework structure type of the zeolite.

Zeolites suitable for use herein include medium or large pore, acidic, hydrophobic zeolites, including without limitation ZSM-5, faujasites, beta, mordenite zeolites or mixtures thereof, having a high silicon to aluminum ratio, such as in the range of 5:1 to 400:1 or 5:1 to 200:1. Medium pore zeolites have a framework structure consisting of 10-membered rings with a pore size of about 0.5-0.6 nm. Large pore zeolites have a framework structure consisting of 12-membered rings with a pore size of about 0.65 to about 0.75 nm. Hydrophobic zeolites generally have Si/AI ratios greater than or equal to about 5, and the hydrophobicity generally increases with increasing Si/AI ratios. Other suitable zeolites include without limitation acidic large pore zeolites such as H-Y with Si/AI in the range of about 2.25 to 5.

Suitable cation exchange resins include, but are not limited to the following: styrene divinylbenzene copolymer-based strong cation exchange resins such as AMBERLYST™ and DOWEX® available from Dow Chemicals (Midland, MI) (for example, DOWEX® Monosphere M-31, AMBERLYST™ 15, AMBERLYST™ BD20); CG resins available from Resintech, Inc. (West Berlin, N.J.); Lewatit resins such as MONOPLUS™ S 100H available from Sybron Chemicals Inc. (Birmingham, N.J.); fluorinated sulfonic acid polymers (these acids are partially or totally fluorinated hydrocarbon polymers containing pendant sulfonic acid groups, which may be partially or totally converted to the salt form) such as NAFION® perfluorinated sulfonic acid polymer, NAFION® Super Acid Catalyst (a bead-form strongly acidic resin which is a copolymer of tetrafluoroethylene and perfluoro-3,6-dioxa-4-methyl-7-octene sulfonyl fluoride, converted to either the proton (H⁺), or the metal salt form) available from DuPont Company (Wilmington, DE).

In an embodiment, the solid acid catalyst is a cation exchange resin that is a sulfonic acid functionalized polymer.

In an embodiment, the solid acid catalyst is a supported acid catalyst. The support for the solid acid catalyst can be any solid substance that is inert under the reaction conditions including, but not limited to, oxides such as silica, alumina, titania, sulfated titania, and compounds thereof and combinations thereof; barium sulfate; calcium carbonate; zirconia; carbons, particularly acid washed carbon; and combinations thereof. Acid washed carbon is a carbon that has been washed with an acid, such as nitric acid, sulfuric acid or acetic acid, to remove impurities. The support can be in the form of powder, granules, pellets, extrudates or the like. The supported acid catalyst can be prepared by depositing the acid catalyst on the support by any number of methods well known to those skilled in the art of catalysis, such as spraying, impregnation, incipient wetness, soaking or physical mixing, followed by drying, calcination, and if necessary, activation through methods such as reduction or oxidation. The loading of the at least one acid catalyst on the at least one support is in the range of 0.1-20 weight percent based on the combined weights of the at least one acid catalyst and the at least one support. Certain acid catalysts perform better at low loadings such as 0.1-5 %, whereas other acid catalysts are more likely to be useful at higher loadings such as 10-20%. In an embodiment, the acid catalyst is an unsupported catalyst having 100% acid catalyst with no support such as, pure zeolites and acidic ion exchange resins.

Examples of supported solid acid catalysts include, but are not limited to, phosphoric acid on silica, NAFION® perfluorinated **2**sulfonic acid polymer on silica, HPAs on silica, sulfated zirconia, and sulfated titania. In the case of NAFION® on silica, a loading of 12.5% is typical of commercial examples.

In another embodiment, the solid acid catalyst comprises a sulfonated divinylbenzene/styrene copolymer, such as AMBERLYST™ 70 and AMBERLYST™ BD20.

In one embodiment, the solid acid catalyst comprises a sulfonated perfluorinated polymer, such as NAFION® supported on silica (SiO₂).

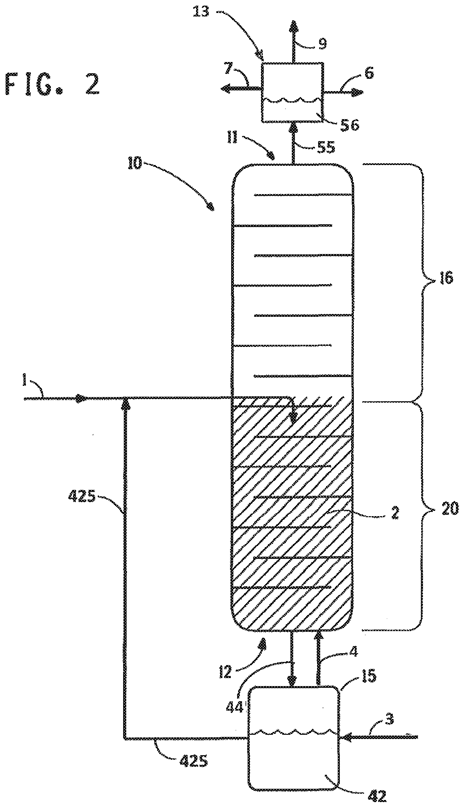

In an embodiment of the present disclosure, the solid acid catalyst **2** is present in the stripping, middle section of the reactive distillation column **10**, which is the reaction zone **20**, as shown in Figures 1 and 2. In an alternative configuration (not shown), the solid acid catalyst **2** is located in a stirred vessel at the bottom of a distillation column similar to the reboiler, which would be a Continuous Stirred-Tank Reactor (CSTR).

The process of making a dialkyl ester of FDCA further comprises, as shown in Figure 1, contacting a feed **1** comprising FDCA and a high boiling solvent with a feed **3** comprising an alcohol in the reaction zone **20** at a temperature in the range of 40 °C to 300 °C and a pressure in the range of 0.0007 MPa (0.1 psi) to 3.4 MPa (500 psi) for a residence time sufficient to produce a mixture comprising a dialkyl ester of FDCA and water.

Suitable high boiling solvent comprises at least one of dimethyl sulfoxide, dimethylacetamide, sulfolane, dimethyl ester of FDCA (FDME), gamma-valerolactone, gamma-butyrolactone, isosorbide dimethyl ether, propylene carbonate, adipic acid, isosorbide, isophorone, ethyl phenyl ether, diphenyl ether, dibenzyl ether, aromatic 200 fluid, butyl phenyl ether, methyl heptyl ketone, ethyl phenyl ketone, 2'-hydroxyacetophenone, decahydronaphthalene, tetrahydronaphthalene, and the like. In an embodiment, the high boiling solvent comprises dimethyl sulfoxide. In another embodiment, the high boiling solvent comprises gamma-valerolactone.

FDCA can be present in the feed comprising FDCA and a high boiling solvent in the range of 1-50 weight % or 1-40 weight % or 5-30 weight %, based on the total weight of the feed.

As shown in Figure 1, the feed stream **1** comprising FDCA is added to the reactive distillation column **10** at a location between a rectifying section **16** and the reaction zone **20** (which is also the stripping section) at a rate that provides sufficient residence time in the reaction zone for conversion of FDCA to dialkyl ester of FDCA. The required residence time is a function of temperature and FDCA concentration and is readily determined by one skilled in the art. In an embodiment, the residence time in the reaction zone is in the range of 0.6-6000 min or 1-600 min or 6-100 min, with a weight hourly space velocity (WHSV) in the range of 0.01 hr⁻¹ to 100 hr⁻¹; or 0.1 hr⁻¹ to 50 hr⁻¹; or 0.1 hr⁻¹ to 10 hr⁻¹; or 1 hr⁻¹ to 10 hr⁻¹.

The feed **1** flows down through the reaction zone **20** and is converted to a mixture comprising a dialkyl ester of FDCA and water.

As shown in Figure 1, the alcohol feed **3** is added to a reboiler **15** along with an inert carrier gas such as nitrogen (not shown). The reboiler **15** is maintained at a temperature high enough to transform the alcohol into vapor form, and vapor stream **4** comprising the alcohol and the carrier gas enters the reaction zone **20** of the reactive distillation column **10** through the bottom **12** of the distillation column. Any suitable alcohol from C₁ to C₆ alcohol may be used and added to the reaction zone through the reboiler. However, C₅ and C₆ alcohol may be added along with the feed **1** at a location between the rectifying section **16** and the reaction zone **20**, due to their high boiling point.

In some embodiments, the alcohol is a C₁ to C₆ alcohol, or a C₁ to C₄ alcohol, or a C₁ to C₂ alcohol. Suitable alcohols can include, for example methanol, ethanol, propanol, butanol, pentanol, hexanol or isomers thereof. The molar ratio of alcohol:FDCA can be in the range of 2.01:1 to 40:1 or 2.2:1 to 40:1 or 2.5:1 to 40:1 or 3:1 to 40:1 or 4:1 to 40:1 or 8:1 to 40:1 or 10:1 to 40:1 or 15:1 to 40:1 or 20:1 to 40:1 or 25:1 to 40:1 or 30:1 to 40:1. In some embodiments, the alcohol is methanol and the ester of FDCA is FDME.

As used herein, the temperature refers to the temperature of the reaction zone. The reaction zone can be at a temperature of at least 40°C or at least 80 °C, or at least 100°C, at least 150 °C, or less than 300°C, or less than 275 °C, or less than 250 °C, or less than 200 °C.

The reaction zone can be at a pressure of at least 0.0007 MPa (0.1 psi), or at least 0.01 MPa, or at least 0.05 MPa, or at least 1 MPa or less than 3.4 MPa (500 psi), or less than 2.5 MPa, or less than 2 MPa, or less than 1.5 MPa. In an embodiment, the reaction is carried at about atmospheric pressure.

Upon esterification of the FDCA in the feed to dialkyl ester of FDCA in the reaction zone, the as-produced dialkyl ester of FDCA flows down and is collected from the bottom of the distillation column and water and methanol are drawn at the top of the distillation column.

The process of making a dialkyl ester of FDCA, as shown in Figure 1 also comprises removing a vapor mixture **5** comprising the alcohol and water from the top **11** of the reactive distillation column **10.** As the reaction proceeds, a vapor mixture **5** is removed from the reaction zone **20** via reflux through reactive distillation column **10**, and at least partially condensed in a reflux condenser **13** to provide a liquid phase **8** comprising the alcohol and water. From the reflux condenser, vapor phase **9** comprising the alcohol can be recycled to the reaction zone (not shown) through the bottom **12** of the reactive distillation column **10,** or can be released from the process. A liquid phase **6** comprising the alcohol and water from reflux condenser **13** can be removed from the process. The use of staging in the distillation process allows more efficient stripping of water away from the reaction. This increases the yield of dialkyl ester of FDCA by driving the reaction toward completion and by minimizing formation of byproducts.

The process as shown in Figure 1 further comprises collecting a product stream **41** from the bottom **12** of the reactive distillation column **10.** The product stream **41** comprises the dialkyl ester of FDCA, the high boiling solvent, and optionally one or more of a monoalkyl ester of FDCA, any unreacted FDCA, and any other high boilers, such as humins. The product stream **41** enters the reboiler **15,** as shown in Figure 1, in which the product stream is mixed with alcohol feed **3** and the carrier gas to form mixture **42.** Liquid stream **43**, comprising the dialkyl ester of FDCA, the high boiling solvent, and optionally one or more of a monoalkyl ester of FDCA, any unreacted FDCA, and any high boilers such as humins, leaves reboiler **15.** Any high boilers such as humins may be removed from stream **43,** for example by filtration (not shown). In Figure 1, stream **43** is shown entering boiler 45, in which the dialkyl ester of FDCA is separated as stream **7** from the high boiling solvent. Residual product stream **415** comprising the high boiling solvent, and optionally one or more of a monoalkyl ester of FDCA, and any unreacted FDCA can be disposed of, or at least a portion can be concentrated by evaporation (not shown) and combined with feed 1 and returned to reactive distillation column **10**, as shown in Figure 1.

In an embodiment of the process of making a dialkyl ester of FDCA, as shown in Figure 2, the FDCA feed **1** and the alcohol feed **3** are added to a reactive distillation column **10** and reboiler **15**, respectively, as described for Figure 1. Solid acid catalyst **2** is present in the reaction zone **20** of the reactive distillation column. In this embodiment, the process comprises removing a vapor mixture **55** comprising the dialkyl ester of FDCA, the alcohol, water, and optionally a monoalkyl ester of FDCA from the top **11** of the reactive distillation column **10**, and collecting a mixture **44** comprising the high boiling solvent, and optionally one or more of a monoalkyl ester of FDCA, any unreacted FDCA, and any other high boilers, such as humins from the bottom **12** of the reactive distillation column **10.** The process can further comprise at least partially condensing vapor mixture 55 in reflux condenser **13** to provide a liquid phase **56**, and separating the dialkyl ester of FDCA as stream **7**, the alcohol as vapor phase **9**, and water as liquid phase **6.** The process may also comprise removing high boilers such as humins from a stream **425** leaving the reboiler **15**, for example by filtration (not shown), wherein residual product stream **425** comprises the high boiling solvent, and optionally one or more of a monoalkyl ester of FDCA and any unreacted FDCA. Residual product stream **425** can be disposed of, or alternatively at least a portion can be concentrated by evaporation (not shown), optionally combined with feed stream **1**, and returned to reactive distillation column **10**, as shown in Figure 2.

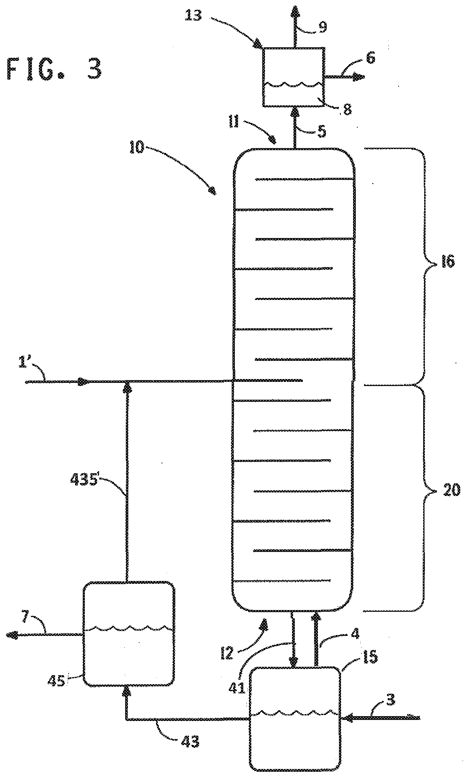

Also disclosed herein is another embodiment of the process of making a dialkyl ester of FDCA, as shown in Figure 3, comprising providing a reactive distillation column **10** comprising a top **11**, a bottom **12**, a reaction zone **20** in between the top **11** and the bottom **12.** In this embodiment, no solid acid catalyst is contained in the reaction zone of the reactive distillation column. A feed stream **1'** comprising FDCA, a high boiling solvent, and optionally a homogeneous catalyst is added to the reactive distillation column **10** at a location between a rectifying section **16** and a reaction zone **20.** An alcohol feed **3** is added to a reboiler **15** along with an inert carrier gas such as nitrogen (not shown). The reboiler **15** is maintained a temperature high enough to transform the alcohol into vapor form, and vapor stream **4** comprising the alcohol and the carrier gas enters the reaction zone of the reactive distillation column through the bottom of the distillation column. The FDCA is contacted with the alcohol in the reaction zone **20** in the absence of a solid acid catalyst at a temperature and a pressure as disclosed hereinabove for a residence time sufficient to produce a mixture comprising a dialkyl ester of FDCA, water and optionally, a monoalkyl ester of FDCA.

As shown in Figure 3, the process also comprises removing a vapor mixture **5** comprising the alcohol and water from the top **11** of the reactive distillation column **10**, and collecting a product stream **41** comprising the dialkyl ester of FDCA, the high boiling solvent, and optionally one or more of the homogeneous catalyst, a monoalkyl ester of FDCA, any unreacted FDCA, and any other high boilers from the bottom **12** of the reactive distillation column **10.** Vapor mixture **5** is at least partially condensed in a reflux condenser **13** to provide a liquid phase **8** comprising the alcohol and water. From the reflux condenser, vapor phase **9** comprising the alcohol can be recycled to the reaction zone (not shown) through the bottom **12** of the reactive distillation column **10**, or can be released from the process. A liquid phase **6** comprising the alcohol and water from reflux condenser **13** can be removed from the process. Product stream **41** is collected from the bottom **12** of the reactive distillation column into reboiler **15.** Liquid stream **43**, comprising the dialkyl ester of FDCA, the high boiling solvent, and optionally one or more of a monoalkyl ester of FDCA, any unreacted FDCA, and any high boilers such as humins is removed from reboiler **15.** Any high boilers such as humins may be removed from stream **43** by filtration (not shown). In Figure 3, stream **43** is shown entering boiler **45**, in which the dialkyl ester of FDCA is separated as stream **7** and a residual product stream **435** comprising the high boiling solvent, and optionally one or more of the homogeneous catalyst, a monoalkyl ester of FDCA, and any unreacted FDCA is produced. The residual product stream can be disposed of, or alternatively at least a portion can be concentrated by evaporation (not shown), combined with feed **1'**, and returned to reactive distillation column **10.**

Suitable homogeneous acid catalyst includes, but is not limited to cobalt (II) acetate, iron (II) chloride, iron (III) chloride, iron (II) sulfate, iron (III) sulfate, iron (II) nitrate, iron (III) nitrate, iron (II) oxide, iron (III) oxide, iron (II) sulfide, iron (III) sulfide, iron (II) acetate, iron (III) acetate, magnesium (II) acetate, magnesium (II) hydroxide, manganese (II) acetate, phosphoric acid, sulfuric acid, zinc (II) acetate, zinc stearate or a combination thereof. Additionally, suitable homogeneous catalysts also include p-toulene sulfonic acid, triflic acid, scandium triflate, hafnium triflate, yttrium triflate, and ytterbium triflate.

The homogeneous acid catalyst can be present in the high boiling solvent at about 0.01 weight percent to about 20 weight percent based on the initial amount of the reactant FDCA. In some embodiments, the acid is present in the high boiling solvent at a weight percentage between and optionally including any two of the following values: 0.01, 0.05, 0.10, 0.15, 0.20, 0.50, 1.0, 1.5, 2.0, 3.0, 4.0, 5.0, 6.0, 7.0, 8.0, 9.0, 10, 11, 12, 13, 14, 15, 16, 17, 18, 19, 20 weight percent. In an embodiment, the acid is present in the high boiling solvent at about 0.01 weight percent to about 10 weight percent. In an embodiment, the acid is present in the solvent at about 0.10 weight percent to about 5 weight percent. The optimal amount of acid catalyst will be affected by what specific high boiling solvent is used and is readily determined by one skilled in the art.

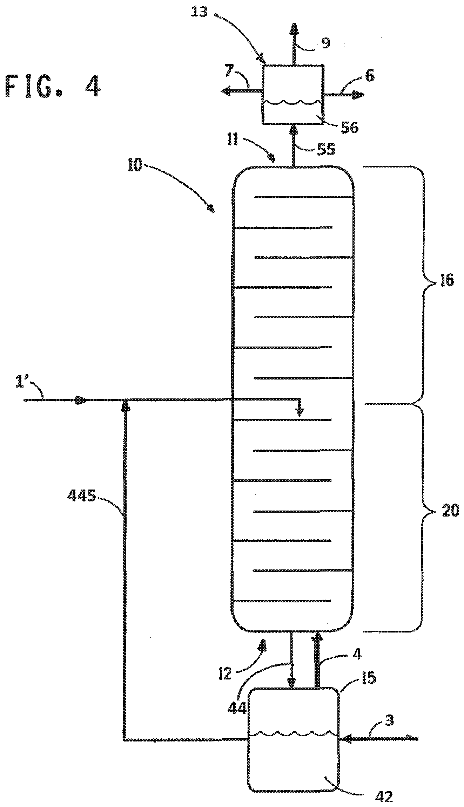

In another embodiment of the process, as shown in Figure 4, no solid catalyst is contained in the reaction zone of the reactive distillation column **10.** A feed stream **1'** comprising FDCA, a high boiling solvent, and optionally a homogeneous catalyst is added to the reactive distillation column **10** as described for Figure 3, as is a feed stream **3** comprising the alcohol. The process comprises removing a vapor mixture **55** comprising the dialkyl ester of FDCA, the alcohol, water, and optionally a monoalkyl ester of FDCA from the top **11** of the reactive distillation column **10** and collecting a mixture **44** comprising the high boiling solvent, and optionally one or more of the homogeneous catalyst, the monoalkyl ester of FDCA, any unreacted FDCA, and any high boilers such as humins from the bottom **12** of the reactive distillation column **20.** The process can further comprise at least partially condensing vapor mixture **55** in reflux condenser **13** to provide a liquid phase **56**, and separating the dialkyl ester of FDCA as stream **7**, the alcohol as vapor phase **9**, and water as liquid phase 6. The process may also comprise removing high boilers such as humins from a stream **445** leaving the reboiler **15**, for example by filtration (not shown), wherein stream **445** comprises the high boiling solvent, and optionally one or more of the homogeneous catalyst, the monoalkyl ester of FDCA, and any unreacted FDCA. Stream **445** can be disposed of, or alternatively at least a portion can be concentrated by evaporation (not shown), optionally combined with feed stream **1'** as shown, and returned to reactive distillation column **10.**

In any of the above disclosed processes, the step of separating the dialkyl ester of FDCA from the product stream and/or the vapor mixture can produce a dialkyl ester of FDCA containing less than 50 parts per million (ppm) of any one of the impurities, for example as determined by HPLC analysis. For example, the dialkyl ester of FDCA can contain less than 50 ppm of the alkyl ester of 5-formylfuran-2-carboxylic acid, less than 50 ppm of the monoalkyl ester of 2,5-furan dicarboxylic acid and/or less than 50 ppm FDCA. In other embodiments, the dialkyl ester of FDCA can contain less than 25 ppm of the alkyl ester of 5-formylfuran-2-carboxylic acid, less than 25 ppm of the monoalkyl ester of 2,5-furan dicarboxylic acid and/or less than 25 ppm FDCA. In still further embodiments, the alkyl ester of FDCA from step c) can contain less than 10 ppm of the alkyl ester of 5-formylfuran-2-carboxylic acid, less than 10 ppm the monoalkyl ester of 2,5-furan dicarboxylic acid and/or less than 10 ppm FDCA. Any monoalkyl ester of 2,5-furan dicarboxylic acid or FDCA remaining from the separation step e) can be recycled back to step b).

Unless otherwise defined, all technical and scientific terms used herein have the same meaning as commonly understood by one of ordinary skill in the art to which this invention belongs. Although methods and materials similar or equivalent to those described herein can be used in the practice or testing of embodiments of the disclosed compositions, suitable methods and materials are described below. In case of conflict, the present specification, including definitions, will control. In addition, the materials, methods, and examples are illustrative only and not intended to be limiting.

In the foregoing specification, the concepts have been disclosed with reference to specific embodiments. However, one of ordinary skill in the art appreciates that various modifications and changes can be made without departing from the scope of the invention as set forth in the claims below.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all embodiments.

### EXAMPLES

The present disclosure is further exemplified in the following Examples. It should be understood that these Examples, while indicating certain preferred aspects herein, are given by way of illustration only. From the above discussion and these Examples, one skilled in the art can ascertain the essential characteristics of the disclosed embodiments, and without departing from the spirit and scope thereof, can make various changes and modifications to adapt the disclosed embodiments to various uses and conditions.

The following abbreviations are used in the examples: "°C" means degrees Celsius; "wt.%" means weight percent; "g" means gram; "min" means minute(s); "µL" means microliter; "ppm" means microgram per gram, "µm" means micrometer; "mL" means milliliter; "mm" means millimeter and "mL/min" means milliliter per minute; "seem" means standard cubic centimeters per minute, "slpm" mean standard liter per minute; "HMF" means 5-(hydroxymethyl)furfural, "DMF" means dimethylformamide, "DMSO" means dimethyl sulfoxide, "FDCA" means 2,5-furandicarboxylic acid, "FDME" means dimethyl-furan-2,5-dicarboxylate, "FDMME" means furan dicarboxylic acid mono methyl ester, "HPLC" means high pressure liquid chromatography.

### Test/General Methods

### HPLC Analysis

HPLC analysis was used as one means to measure the FDCA, FDMME, and FDME contents of the product mixture. An Agilent 1200 series HPLC equipped with a Zorbax SB-Aq column (4.6 mm X 250 mm, 5 µm) and photodiode array detector was used for the analysis of the reaction samples. The wavelength used to monitor the reaction was 280 nm. The HPLC separation of FDME, FDCA, and FDMME was achieved using a gradient method with a 1.0 mL/min flow rate combining two mobile phases: Mobile Phase A: 0.5% v/v TFA in water and Mobile Phase B: acetonitrile. The column was held at 60 °C and 2 µL injections of samples were performed. Analyzed samples were diluted to <0.1 wt% for components of interest in a 50:50 (v/v) acetonitrile/isopropanol solvent. The solvent composition and flow rates used for the gradient method are given in Table 1 with linear changes occurring over the corresponding step whenever the composition changes.

**Table 1: Gradient program for HPLC**

| Step | Start time (min) | Volume % Mobile Phase B, at Beginning of Step | Volume % Mobile Phase B, at End of Step |
|---|---|---|---|
| 1 | 0.0 | 0 | 0 |
| 2 | 6.0 | 0 | 80 |
| 3 | 20.0 | 80 | 80 |
| 4 | 25.0 | 80 | 0 |
| 5 | 25.1 | 0 | 0 |
| 6 | 30.0 | 0 | 0 |

Retention times were obtained by injecting analytical standards of each component onto the HPLC. The amount of the analyte in weight percent was typically determined by injection of two or more injections from a given prepared solution and averaging the area measured for the component using the OpenLAB CDS C.01.05 software. The solution analyzed by HPLC was generated by dilution of a measured mass of the reaction sample with a quantified mass of 50:50 (v/v) acetonitrile/isopropanol solvent. Quantification was performed by comparing the areas determined in the OpenLAB software to a linear external calibration curve at five or more starting material concentrations. Typical R² values for the fit of such linear calibration curves were in excess of 0.9997.

While the presented HPLC method was used for this analysis, it should be understood that any HPLC method that can discriminate between products, starting materials, intermediates, impurities, and solvent can be used for this analysis. It should also be understood that while HPLC was used as a method of analysis in this work, other techniques such as gas chromatography could also be optionally used for quantification when employing appropriate derivatization and calibration as necessary.

### Materials Used

All commercial reagents were used as received. DMSO was obtained from Sigma-Aldrich. Methanol (catalog # MX-0472-6) was obtained from EMD Millipore (Billerica, MA). Acetonitrile (catalog # A955-1) and Isopropanol (catalog # A4641L1) were obtained from Fisher Scientific (Pittsburgh, PA). FDCA (> 99.0% purity) was obtained from Sarchem Laboratories, Inc (Farmingdale, NJ). Ethylene glycol (BDH 2033) was provided by VWR International (Radnor, DE).

Amberlyst™ BD20 catalyst in bead form was received from Dow Chemical Company (Newark, DE).

Glass beads (Catalog No. CG-1101-01) of 2 mm diameter were obtained from Chemglass Inc (Vineland, NJ).

### EXAMPLE 1

### Esterification of FDCA to FDME via Reactive Distillation with Solid Acid Catalyst

Esterification of FDCA to FDME was carried out in a reactive distillation unit consisting of a jacketed glass reactor having a length of 21.6 cm (8.5 inches) and an outer diameter of 3.5 cm (1.38 inches). The middle section of the glass reactor was filled with about 50 gm (WHSV = 0.1 hr⁻¹) of Amberlyst™ BD20 catalyst and the top and the bottom portions of the reactor were packed with glass beads. A stainless steel mesh was placed below the glass beads in the bottom portion of the reactor to support the catalyst bed and the glass beads. The catalyst bed temperature was monitored using a thermocouple and was maintained at about 105 °C using circulating oil in the outer jacket surrounding the glass reactor. The top of the glass reactor was connected to a condensor maintained at a temperature of 10 °C using a circulating coolant containing 1:1 mixture by weight of ethylene glycol and water.

A feed consisting of 10 wt.% of 2,5-furan dicarboxylic acid in 90 wt.% of high boiling solvent, dimethyl sulfoxide, was fed at a constant feed rate of 1 mL/min to the top part of the reactor above the catalyst bed, using an HPLC pump. Methanol was fed at 1 mL/min to the catalyst bed forming the reaction zone through one of the three necks of a round-bottom glass flask connected to the bottom of glass reactor, which was kept at a temperature of 100-120 °C. Nitrogen gas was also introduced to the reactor through one of the necks of the round-bottom glass flask.

The feed and methanol flowing counter current to each other reacted in the presence of the Amberlyst™BD20 catalyst in the reaction zone maintained at 105 °C and under atmospheric pressure to form esterified products of FDCA - including FDME and FDMME and water as a byproduct. The product stream comprising FDME and FDMME, along with any unreacted FDCA and DMSO were collected from the bottom of the glass reactor in the round-bottom glass flask and a vapor mixture comprising water vapor and unreacted methanol were collected in the condenser located at the top of the reactor.

The product stream was collected in a sample vial, weighed and analyzed by HPLC. Table 2 below shows the analysis of the product obtained at the end of 4 hour run. The data are reported for the steady state conditions achieved in the reactor.

**Table 2 - Product obtained at the end of 4 hour esterification reaction using Amberlyst™ BD20 catalyst in a reactive distillation reactor.**

| Sample | Time (min) | FDCA | FDMME | FDME |
|---|---|---|---|---|
| 1.1 | 240 | 54.5% | 35.0% | 10.5% |

As seen from the data in Table 2, use of the Amberlyst™ BD20 catalyst resulted in the esterification of FDCA to FDME and FDMME. Thus, the reactive distillation process with solid acid catalyst and high boiling solvent can be used for the esterification of FDCA to FDME.

## Claims

1. A process comprising:
a) providing a reactive distillation column comprising a top, a bottom, and a reaction zone in between the top and the bottom, and a solid acid catalyst situated in the reaction zone;
b) contacting a feed comprising 2,5-furan dicarboxylic acid and a high boiling solvent with an alcohol in the reaction zone at a temperature in the range of 40 °C to 300 °C and a pressure in the range of 0.0007 MPa to 3.4 MPa for a residence time sufficient to produce a mixture comprising a dialkyl ester of 2,5-furan dicarboxylic acid and water;
c) removing a vapor mixture comprising the alcohol and water from the top of the reactive distillation column;
d) collecting a product stream comprising the dialkyl ester of 2,5-furan dicarboxylic acid, the high boiling solvent, and optionally one or more of a monoalkyl ester of 2,5-furan dicarboxylic acid, any unreacted 2,5-furan dicarboxylic acid and any other high boilers from the bottom of the reactive distillation column; and
e) separating the dialkyl ester of 2,5-furan dicarboxylic acid from the product stream using distillation.

2. The process of claim 1, wherein the temperature in the reaction zone is in the range of 40°C to 150°C.

3. The process of claim 1 wherein the high boiling solvent comprises at least one of dimethyl sulfoxide, dimethylacetamide, sulfolane, dimethyl ester of 2,5-furan dicarboxylic acid, gamma-valerolactone, gamma-butyrolactone, isosorbide dimethyl ether, propylene carbonate, adipic acid, isosorbide, isophorone, ethyl phenyl ether, diphenyl ether, dibenzyl ether, aromatic 200 fluid, butyl phenyl ether, methyl heptyl ketone, ethyl phenyl ketone, 2'-hydroxyacetophenone, decahydronaphthalene, or tetrahydronaphthalene.

4. The process of claim 1, wherein the alcohol comprises C₁ to C₆ alcohol.

5. The process of claim 1, wherein the alcohol is methanol.

6. The process of claim 1, wherein alcohol is fed with a carrier gas from the bottom of the reactive distillation column and the feed comprising 2,5-furan dicarboxylic acid and the high boiling solvent is fed from the top of the reactive distillation column.

7. The process of claim 1, wherein the acid catalyst comprises a heterogeneous heteropolyacid, a salt of a heterogeneous heteropolyacid, a natural or synthetic mineral, an ion- exchange resin, a metal oxide, a mixed metal oxide, a metal sulfide, a metal sulfate, a metal sulfonate, sulfated titania, sulfated zirconia, a metal nitrate, a metal phosphate, a metal phosphonate, a metal molybdate, a metal tungstate, a metal borate, sulfonic-acid-functionalized polymer, or a combination of any of these.

8. The process according to claim 1, wherein the acid catalyst comprises a zeolite.

9. The process according to claim 8, wherein the acid catalyst is a medium or large pore, acidic, hydrophobic zeolite.

10. The process according to claim 8, wherein the zeolite comprises ZSM-5, faujasite, beta zeolite, mordenite, or a combination of any of these.

11. A process comprising:
a) providing a reactive distillation column comprising a top, a bottom, and a reaction zone in between the top and the bottom, and a solid acid catalyst situated in the reaction zone;
b) contacting a feed comprising 2,5-furan dicarboxylic acid and a high boiling solvent with an alcohol in the reaction zone at a temperature in the range of 40 °C to 300 °C and a pressure in the range of 0.0007 MPa to 3.4 MPa for a residence time sufficient to produce a vapor mixture of a dialkyl ester of 2,5-furan dicarboxylic acid, water and optionally, a monoalkyl ester of 2,5-furan dicarboxylic acid;
c) removing a vapor mixture comprising the dialkyl ester of 2,5-furan dicarboxylic acid, the alcohol, water, and optionally the monoalkyl ester of 2,5-furan dicarboxylic acid from the top of the reactive distillation column;
d) collecting the high boiling solvent, optionally one or more of a monoalkyl ester of 2,5-furan dicarboxylic acid, any unreacted 2,5-furan dicarboxylic acid, and any other high boilers from the bottom of the reactive distillation column; and
e) separating the dialkyl ester of 2,5-furan dicarboxylic acid from the vapor mixture using distillation.

12. A process according to claim 11, wherein the temperature in the reaction zone is in the range of 40°C to 150°C.

13. A process comprising:
a) providing a reactive distillation column comprising a top, a bottom, and a reaction zone in between the top and the bottom;
b) contacting a feed comprising 2,5-furan dicarboxylic acid, a high boiling solvent, and an optional homogeneous catalyst with an alcohol in the reaction zone in the absence of a solid acid catalyst at a temperature in the range of 150 °C to 300 °C and a pressure in the range of 0.0007 MPa to 3.4 MPa for a residence time sufficient to produce a mixture comprising a dialkyl ester of 2,5-furan dicarboxylic acid, water and, optionally, a monoalkyl ester of 2,5-furan dicarboxylic acid;
c) removing a vapor mixture comprising the alcohol and water from the top of the reactive distillation column;
d) collecting a product stream comprising the dialkyl ester of 2,5-furan dicarboxylic acid, the high boiling solvent, and optionally one or more of the homogeneous catalyst, the monoalkyl ester of 2,5-furan dicarboxylic acid, any unreacted 2,5-furan dicarboxylic acid and any other high boilers from the bottom of the reactive distillation column; and
e) separating the dialkyl ester of 2,5-furan dicarboxylic acid from the product stream using distillation.

14. The process of claim 13, wherein the homogeneous catalyst is at least one of cobalt (II) acetate, iron (II) chloride, iron (III) chloride, iron (II) sulfate, iron (III) sulfate, iron (II) nitrate, iron (III) nitrate, iron (II) oxide, iron (III) oxide, iron (II) sulfide, iron (III) sulfide, iron (II) acetate, iron (III) acetate, magnesium (II) acetate, magnesium (II) hydroxide, manganese (II) acetate, phosphoric acid, sulfuric acid, zinc (II) acetate, zinc stearate, or a combination thereof.

15. A process comprising:
a) providing a reactive distillation column comprising a top, a bottom, and a reaction zone in between the top and the bottom;
b) contacting a feed comprising 2,5-furan dicarboxylic acid, a high boiling solvent, and an optional homogeneous catalyst with an alcohol in the reaction zone in the absence of a solid acid catalyst at a temperature in the range of 150 °C to 300 °C and a pressure in the range of 0.0007 MPa to 3.4 MPa for a residence time sufficient to produce a mixture comprising a dialkyl ester of 2,5-furan dicarboxylic acid and water;
c) removing a vapor mixture comprising the dialkyl ester of 2,5-furan dicarboxylic acid, the alcohol, water, and optionally a monoalkyl ester of 2,5-furan dicarboxylic acid from the top of the reactive distillation column;
d) collecting the high boiling solvent, and optionally one or more of the homogeneous catalyst, the monoalkyl ester of 2,5-furan dicarboxylic acid, any unreacted 2,5-furan dicarboxylic acid and high boilers from the bottom of the reactive distillation column; and
e) separating the dialkyl ester of 2,5-furan dicarboxylic acid from the vapor mixture using distillation.

## Patentansprüche

1. Verfahren, umfassend:
a) Bereitstellen einer Reaktiv-Destillationskolonne, umfassend einen Kopf, einen Boden und eine Reaktionszone zwischen dem Kopf und dem Boden und einen festen sauren Katalysator, der sich in der Reaktionszone befindet;
b) Inkontaktbringen einer 2,5-Furandicarbonsäure und ein hochsiedendes Lösemittel umfassenden Beschickung mit einem Alkohol in der Reaktionszone bei einer Temperatur im Bereich von 40 °C bis 300 °C und einem Druck im Bereich von 0,0007 MPa bis 3,4 MPa für eine Verweilzeit, die ausreichend ist, um eine Mischung zu erzeugen, die einen Dialkylester von 2,5-Furandicarbonsäure und Wasser umfasst;
c) Entfernen eines den Alkohol und das Wasser umfassenden Dampfgemisches aus dem Kopf der Reaktiv-Destillationskolonne;
d) Sammeln eines Produktstroms, umfassend den Dialkylester von 2,5-Furandicarbonsäure, das hochsiedende Lösemittel und wahlweise eines oder mehrerer eines Monoalkylesters von 2,5-Furandicarbonsäure, etwaiger nicht umgesetzter 2,5-Furandicarbonsäure und etwaiger anderer Hochsieder aus dem Boden der Reaktiv-Destillationskolonne; und
e) Abtrennen des Dialkylesters der 2,5-Furandicarbonsäure aus dem Produktstrom durch Anwendung von Destillation.

2. Verfahren nach Anspruch 1, wobei die Temperatur in der Reaktionszone im Bereich von 40 °C bis 150 °C liegt.

3. Verfahren nach Anspruch 1, wobei das hochsiedende Lösemittel mindestens eines der folgenden umfasst: Dimethylsulfoxid, Dimethylacetamid, Sulfolan, Dimethylester von 2,5-Furandicarbonsäure, gamma-Valerolacton, gamma-Butyrolacton, Isosorbiddimethylether, Propylencarbonat, Adipinsäure, Isosorbid, Isophoron, Ethylphenylether, Diphenylether, Dibenzylether, Aromatic 200 Fluid, Butylphenylether, Methylheptylketon, Ethylphenylketon, 2'-Hydroxyacetophenon, Decahydronaphthalin oder Tetrahydronaphthalin.

4. Verfahren nach Anspruch 1, wobei der Alkohol C₁-bis C₆-Alkohol umfasst.

5. Verfahren nach Anspruch 1, wobei der Alkohol Methanol ist.

6. Verfahren nach Anspruch 1, wobei Alkohol mit einem Trägergas aus dem Boden der Reaktiv-Destillationskolonne eingespeist wird und die Einspeisung, die 2,5-Furandicarbonsäure und das hochsiedende Lösemittel umfasst, aus dem Kopf der Reaktiv-Destillationskolonne eingespeist wird.

7. Verfahren nach Anspruch 1, wobei der saure Katalysator eine heterogene Heteropolysäure, ein Salz einer heterogenen Heteropolysäure, ein natürliches oder synthetisches Mineral, ein Ionenaustauscherharz, ein Metalloxid, ein gemischtes Metalloxid, ein Metallsulfid, ein Metallsulfat, ein Metallsulfonat, sulfatiertes Titandioxid, sulfatiertes Zirkoniumdioxid, ein Metallnitrat, ein Metallphosphat, ein Metallphosphonat, ein Metallmolybdat, ein Metallwolframat, ein Metallborat, ein mit Sulfonsäure funktionalisiertes Polymer oder eine Kombination eines beliebigen davon umfasst.

8. Verfahren nach Anspruch 1, wobei der saure Katalysator einen Zeolith umfasst.

9. Verfahren nach Anspruch 8, wobei der saure Katalysator ein mittel- oder großporiger, saurer, hydrophober Zeolith ist.

10. Verfahren nach Anspruch 8, wobei der Zeolith ZSM-5, Faujasit, Beta-Zeolith, Mordenit oder eine Kombination beliebiger davon umfasst.

11. Verfahren, umfassend:
a) Bereitstellen einer Reaktiv-Destillationskolonne, die einen Kopf, einen Boden und eine Reaktionszone zwischen dem Kopf und dem Boden und einen festen sauren Katalysator umfasst, der sich in der Reaktionszone befindet;
b) Inkontaktbringen einer Beschickung, die 2,5-Furandicarbonsäure und ein hochsiedendes Lösemittel umfasst, mit einem Alkohol in der Reaktionszone bei einer Temperatur im Bereich von 40 °C bis 300 °C und einem Druck im Bereich von 0,0007 MPa bis 3,4 MPa für eine Verweilzeit, die ausreichend ist, um ein Dampfgemisch aus einem Dialkylester von 2,5-Furandicarbonsäure, Wasser und wahlweise einem Monoalkylester von 2,5-Furandicarbonsäure zu erzeugen;
c) Entfernen eines Dampfgemisches, das den Dialkylester von 2,5-Furandicarbonsäure, den Alkohol, Wasser und wahlweise den Monoalkylester von 2,5-Furandicarbonsäure umfasst, aus dem Kopf der Reaktiv-Destillationskolonne;
d) Sammeln des hochsiedenden Lösemittels, wahlweise eines oder mehrerer eines Monoalkylesters von 2,5-Furandicarbonsäure, etwaiger nicht umgesetzter 2,5-Furandicarbonsäure und etwaiger anderer Hochsieder aus dem Boden der Reaktiv-Destillationskolonne; und
e) Abtrennen des Dialkylesters der 2,5-Furandicarbonsäure aus dem Dampfgemisch unter Anwendung von Destillation.

12. Verfahren nach Anspruch 11, wobei die Temperatur in der Reaktionszone im Bereich von 40°C bis 150 °C liegt.

13. Verfahren, umfassend:
a) Bereitstellen einer Reaktiv-Destillationskolonne, die einen Kopf, einen Boden und eine Reaktionszone zwischen dem Kopf und dem Boden umfasst;
b) Inkontaktbringen einer Beschickung, die 2,5-Furandicarbonsäure, ein hochsiedendes Lösemittel und einen wahlweise homogenen Katalysator umfasst, mit einem Alkohol in der Reaktionszone in Abwesenheit eines festen sauren Katalysators bei einer Temperatur im Bereich von 150 °C bis 300°C und einem Druck im Bereich von 0,0007 MPa bis 3,4 MPa für eine Verweilzeit, die ausreichend ist, um eine Mischung zu erzeugen, die einen Dialkylester von 2,5-Furandicarbonsäure, Wasser und wahlweise einen Monoalkylester von 2,5-Furandicarbonsäure umfasst;
c) Entfernen eines den Alkohol und das Wasser umfassenden Dampfgemisches aus dem Kopf der Reaktiv-Destillationskolonne;
d) Sammeln eines Produktstroms, umfassend den Dialkylester von 2,5-Furandicarbonsäure, das hochsiedende Lösemittel und wahlweise eines oder mehrere des homogenen Katalysators, des Monoalkylesters von 2,5-Furandicarbonsäure, etwaiger nicht umgesetzter 2, 5-Furandicarbonsäure und etwaiger anderer Hochsieder aus dem Boden der Reaktiv-Destillationskolonne; und
e) Abtrennen des Dialkylesters der 2,5-Furandicarbonsäure aus dem Produktstrom unter Anwendung von Destillation.

14. Verfahren nach Anspruch 13, wobei der homogene Katalysator mindestens eines ist aus den folgenden: Cobalt(II)-acetat, Eisen(II)-chlorid, Eisen(III)-chlorid, Eisen(II)-sulfat, Eisen(III)-sulfat, Eisen(II)-nitrat, Eisen(III)-nitrat, Eisen(II)-oxid, Eisen(III)-oxid, Eisen(II)-sulfid, Eisen(III)-sulfid, Eisen(II)-acetat, Eisen(III)-acetat, Magnesium(II)-acetat, Magnesium(II)-hydroxid, Mangan(II)-acetat, Phosphorsäure, Schwefelsäure, Zink(II)-acetat, Zinkstearat oder eine Kombination davon.

15. Verfahren, umfassend:
a) Bereitstellen einer Reaktiv-Destillationskolonne, die einen Kopf, einen Boden und eine Reaktionszone zwischen dem Kopf und dem Boden umfasst;
b) Inkontaktbringen einer Beschickung, die 2,5-Furandicarbonsäure, ein hochsiedendes Lösemittel und einen wahlweise homogenen Katalysator umfasst, mit einem Alkohol in der Reaktionszone in Abwesenheit eines festen sauren Katalysators bei einer Temperatur im Bereich von 150 °C bis 300 °C und einem Druck im Bereich von 0,0007 MPa bis 3,4 MPa für eine Verweilzeit, die ausreichend ist, um eine Mischung zu erzeugen, die einen Dialkylester von 2,5-Furandicarbonsäure und Wasser umfasst;
c) Entfernen eines Dampfgemisches, das den Dialkylester von 2,5-Furandicarbonsäure, den Alkohol, Wasser und wahlweise einen Monoalkylester von 2,5-Furandicarbonsäure umfasst, aus dem Kopf der Reaktiv-Destillationskolonne;
d) Sammeln des hochsiedenden Lösemittels und wahlweise eines oder mehrerer des homogenen Katalysators, des Monoalkylesters von 2,5-Furandicarbonsäure, etwaiger nicht umgesetzter 2,5-Furandicarbonsäure und etwaiger Hochsieder aus dem Boden der Reaktiv-Destillationskolonne; und
e) Abtrennen des Dialkylesters von 2,5-Furandicarbonsäure aus dem Dampfgemisch unter Anwendung von Destillation.

## Revendications

1. Procédé comprenant:
a) la fourniture d'une colonne de distillation réactive comprenant un sommet, un bas, et une zone de réaction entre le sommet et le bas, et un catalyseur acide solide situé dans la zone de réaction;
b) la mise en contact d'une charge d'alimentation comprenant de l'acide 2,5-furane dicarboxylique et un solvant à haut point d'ébullition avec un alcool dans la zone de réaction à une température dans la gamme de 40°C à 300°C et une pression dans la gamme de 0,0007 MPa à 3,4 MPa pendant un temps de séjour suffisant pour produire un mélange comprenant un ester dialkylique d'acide 2,5-furane dicarboxylique et de l'eau;
c) l'enlèvement d'un mélange de vapeurs comprenant l'alcool et l'eau par le sommet de la colonne de distillation réactive;
d) la collecte d'un courant de produit comprenant l'ester dialkylique d'acide 2,5-furane dicarboxylique, le solvant à haut point d'ébullition, et optionnellement un ou plusieurs ester monoalkyliques d'acide 2,5-furane dicarboxylique, tout acide 2,5-furane dicarboxylique n'ayant pas réagi et tous autres produits à haut point d'ébullition par le bas de la colonne de distillation réactive; et
e) la séparation de l'ester dialkylique d'acide 2,5-furane dicarboxylique à partir du courant de produits en utilisant une distillation.

2. Procédé selon la revendication 1, dans lequel la température dans la zone de réaction est dans la gamme de 40°C à 150°C.

3. Procédé selon la revendication 1, dans lequel le solvant à haut point d'ébullition comprend au moins l'un parmi le diméthyl sulfoxyde, le diméthylacétamide, le sulfolane, l'ester diméthylique d'acide 2,5-furane dicarboxylique, la gamma-valérolactone, la gamma-butyrolactone, l'éther diméthylique d'isosorbide, le carbonate de propylène, l'acide adipique, l'isosorbide, l'isophorone, l'éther d'éthyle et de phényle, l'éther de diphényle, l'éther de dibenzyle, le fluide aromatique 200, l'éther de butyle et de phényle, la méthyl heptyl cétone, l'éthyl phényl cétone, la 2'-hydroxyacétophénone, le décahydronaphtalène ou le tétrahydronaphtalène.

4. Procédé selon la revendication 1, dans lequel l'alcool comprend un alcool en C₁ à C₆.

5. Procédé selon la revendication 1, dans lequel l'alcool est le méthanol.

6. Procédé selon la revendication 1, dans lequel l'alcool est alimenté avec un gaz porteur depuis le bas de la colonne de distillation réactive et la charge d'alimentation comprenant l'acide 2,5-furane dicarboxylique et le solvant à haut point d'ébullition est alimentée par le sommet de la colonne de distillation réactive.

7. Procédé selon la revendication 1, dans lequel le catalyseur acide comprend un hétéropolyacide hétérogène, un sel d'hétéropolyacide hétérogène, un minéral naturel ou synthétique, une résine échangeuse d'ions, un oxyde de métal, un oxyde de métal mixte, un sulfure de métal, un sulfate de métal, un sulfonate de métal, un dioxyde de titane sulfaté, une zircone sulfatée, un nitrate de métal, un phosphate de métal, un phosphonate de métal, un molybdate de métal, un tungstate de métal, un borate de métal, un polymère d'acide sulfonique fonctionnalisé, ou une combinaison de n'importe lesquels de ceux-ci.

8. Procédé selon la revendication 1, dans lequel le catalyseur acide comprend une zéolite.

9. Procédé selon la revendication 8, dans lequel le catalyseur acide est une zéolite hydrophobe acide à pores moyens ou grands.

10. Procédé selon la revendication 8, dans lequel la zéolite comprend ZSM-5, la faujasite, la bêta zéolite, la mordénite, ou une combinaison de n'importe lesquels de ceux-ci.

11. Procédé comprenant:
a) la fourniture d'une colonne de distillation réactive comprenant un sommet, un bas, et une zone de réaction entre le sommet et le bas, et un catalyseur acide solide situé dans la zone de réaction;
b) la mise en contact d'une charge d'alimentation comprenant de l'acide 2,5-furane dicarboxylique et un solvant à haut point d'ébullition avec un alcool dans la zone de réaction à une température dans la gamme de 40°C à 300°C et une pression dans la gamme de 0,0007 MPa à 3,4 MPa pendant un temps de séjour suffisant pour produire un mélange de vapeurs comprenant un ester dialkylique d'acide 2,5-furane dicarboxylique, de l'eau et optionnellement un ester monoalkylique d'acide 2,5-furane dicarboxylique;
c) l'enlèvement d'un mélange de vapeurs comprenant l'ester dialkylique d'acide 2,5-furane dicarboxylique, l'alcool, l'eau et optionnellement l'ester monoalkylique d'acide 2,5-furane dicarboxylique par le sommet de la colonne de distillation réactive;
d) la collecte du solvant à haut point d'ébullition, optionnellement d'un ou plusieurs parmi un ester monoalkylique d'acide 2,5-furane dicarboxylique, tout acide 2,5-furane dicarboxylique non réagi et tous autres produits à haut point d'ébullition par le bas de la colonne de distillation réactive; et
e) la séparation de l'ester dialkylique d'acide 2,5-furane dicarboxylique à partir du mélange de vapeurs en utilisant une distillation.

12. Procédé selon la revendication 11, dans lequel la température dans la zone de réaction est dans la gamme de 40°C à 150°C.

13. Procédé comprenant:
a) la fourniture d'une colonne de distillation réactive comprenant un sommet, un bas, et une zone de réaction entre le sommet et le bas;
b) la mise en contact d'une charge d'alimentation comprenant de l'acide 2,5-furane dicarboxylique, un solvant à haut point d'ébullition et un catalyseur homogène facultatif avec un alcool dans la zone de réaction en l'absence d'un catalyseur acide solide à une température dans la gamme de 150°C à 300°C et une pression dans la gamme de 0,0007 MPa à 3,4 MPa pendant un temps de séjour suffisant pour produire un mélange comprenant un ester dialkylique d'acide 2,5-furane dicarboxylique, de l'eau et, optionnellement, un ester monoalkylique d'acide 2,5-furane dicarboxylique;
c) l'enlèvement d'un mélange de vapeurs comprenant l'alcool et l'eau par le sommet de la colonne de distillation réactive;
d) la collecte d'un courant de produits comprenant l'ester dialkylique d'acide 2,5-furane dicarboxylique, le solvant à haut point d'ébullition, et optionnellement un ou plusieurs parmi le catalyseur homogène, l'ester monoalkylique d'acide 2,5-furane dicarboxylique, tout acide 2,5-furane dicarboxylique non réagi et tous autres produits à haut point d'ébullition par le bas de la colonne de distillation réactive; et
e) la séparation de l'ester dialkylique d'acide 2,5-furane dicarboxylique à partir du courant de produits en utilisant une distillation.

14. Procédé selon la revendication 13, dans lequel le catalyseur homogène est au moins l'un parmi l'acétate de cobalt (II), le chlorure de fer (II), le chlorure de fer (III), le sulfate de fer (II), le sulfate de fer (III), le nitrate de fer (II), le nitrate de fer (III), l'oxyde de fer (II), l'oxyde de fer (III), le sulfure de fer (II), le sulfure de fer (III), l'acétate de fer (II), l'acétate de fer (III), l'acétate de magnésium (II), l'hydroxyde de magnésium (II), l'acétate de manganèse (II), l'acide phosphorique, l'acide sulfurique, l'acétate de zinc (II), le stéarate de zinc, ou une combinaison de ceux-ci.

15. Procédé comprenant:
a) la fourniture d'une colonne de distillation réactive comprenant un sommet, un bas, et une zone de réaction entre le sommet et le bas;
b) la mise en contact d'une charge d'alimentation comprenant de l'acide 2,5-furane dicarboxylique, un solvant à haut point d'ébullition et un catalyseur homogène facultatif avec un alcool dans la zone de réaction en l'absence d'un catalyseur acide solide à une température dans la gamme de 150°C à 300°C et une pression dans la gamme de 0,0007 MPa à 3,4 MPa pendant un temps de séjour suffisant pour produire un mélange comprenant un ester dialkylique d'acide 2,5-furane dicarboxylique et de l'eau;
c) l'enlèvement d'un mélange de vapeurs comprenant l'ester dialkylique d'acide 2,5-furane dicarboxylique, l'alcool, l'eau et optionnellement un ester monoalkylique d'acide 2,5-furane dicarboxylique par le sommet de la colonne de distillation réactive;
d) la collecte du solvant à haut point d'ébullition, et optionnellement d'un ou plusieurs parmi le catalyseur homogène, l'ester monoalkylique d'acide 2,5-furane dicarboxylique, tout acide 2,5-furane dicarboxylique non réagi et tous autres produits à haut point d'ébullition par le bas de la colonne de distillation réactive; et
e) la séparation de l'ester dialkylique d'acide 2,5-furane dicarboxylique à partir du mélange de vapeurs en utilisant une distillation.
